(19) Europäisches Patentamt
European Patent Office
Office européen des brevets

(11) **EP 4 546 266 A1**

## (12) EUROPEAN PATENT APPLICATION

(43) Date of publication:
**30.04.2025 Bulletin 2025/18**

(21) Application number: **24207754.3**

(22) Date of filing: **21.10.2024**

(51) International Patent Classification (IPC):
**G06T 7/593** (2017.01)

(52) Cooperative Patent Classification (CPC):
**G06T 7/593;** G06T 2200/08; G06T 2207/10081;
G06T 2207/10116; G06T 2207/30101;
G06T 2207/30172

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **23.10.2023 KR 20230142298**

(71) Applicant: **Medipixel, Inc.**
**Seoul 06174 (KR)**

(72) Inventor: **KU, Jong Hoe**
**06174 Seoul (KR)**

(74) Representative: **Studio Torta S.p.A.**
**Via Viotti, 9**
**10121 Torino (IT)**

### (54) DEVICE AND METHOD TO RECONSTRUCT THREE DIMENSIONAL SHAPE OF VESSEL

(57)     An electronic device is disclosed. The electronic device comprises an image acquisition unit configured to acquire medical images captured at a plurality of positions, and a processor configured to generate tree structure data of three-dimensional candidate points corresponding to points along center lines of blood vessel regions of a reference image and a sub-image of the medical images, determine a three-dimensional path based on the tree structure data, determine a three-dimensional diameter for each of the points on the three-dimensional path, and reconstruct the three-dimensional shape of the blood vessels corresponding to the three-dimensional path based on the determined three-dimensional diameter.

FIG. 4

**Description**

**BACKGROUND**

**TECHNICAL FIELD**

The present disclosure relates to a technique for reconstructing a three-dimensional shape of blood vessels.

**DESCRIPTION OF THE RELATED ART**

Angiography is a diagnostic procedure that visualizes blood vessels and conditions using X-rays and is a useful tool for examining vascular diseases. With the angiography useful for the diagnosis of vascular diseases, accurately identifying the size and shape of blood vessels is important for selecting a severity of a disease and an appropriate method of treatment for the disease. In particular, selecting a treatment method requires accurate calculation of indicators related to blood flow, and to this end, it is necessary to quickly and accurately reconstruct the three-dimensional shape of the blood vessels.

The related technology described above has come to possession of the inventors or acquired by the inventors in the process of deriving the description of the present disclosure, and should not necessarily be considered as a publicly known technology disclosed to the public prior to this application.

**SUMMARY**

With the reconstruction of the 3D shape of the blood vessels according to some aspects, it is possible to determine a 3D path of the blood vessels using the tree structure data of the nodes representing the 3D candidate points corresponding to the points along the center lines of two blood vessel images.

With the reconstruction of the 3D shape of the blood vessels according to some aspects, it is possible to prevent a reconstruction error of a severely bent sections caused due to the influence of the force vectors spreading to both sides rather than the force vectors directed inward in the active contour.

With the reconstruction of the 3D shape of the blood vessels according to some aspects, it is possible to prevent a reconstruction error caused due to a plurality of duplicate matching among sections in which the epipolar lines and the center lines of the blood vessels are parallel in the path finding algorithm (e.g., dynamic programming).

However, it is to be noted that the technical problems to be solved by the disclosure are not limited to the those mentioned above, and other technical problems may exist.

According to an aspect of the present disclosure, a method to reconstruct a three-dimensional shape of blood vessels may be implemented by a processor and may comprise acquiring medical images captured at a plurality of positions, generating tree structure data of three-dimensional candidate points corresponding to points along center lines of blood vessel regions of a reference image and a sub-image of the medical images, determining a three-dimensional path based on the tree structure data, determining a three-dimensional diameter for each of the points on the three-dimensional path, and reconstructing the three-dimensional shape of the blood vessels corresponding to the three-dimensional path based on the determined three-dimensional diameter.

According to an aspect of the present disclosure, the generating the tree structure data may include determining a medical image selected from among the plurality of medical images based on at least one of a length of the center line or lesion information as the reference image.

According to an aspect of the present disclosure, the generating the tree structure data may include determining the three-dimensional candidate points corresponding to sub-points along a center line of a segmented blood vessel region in the sub-image, for each of reference points along a center line of the segmented blood vessel region in the reference image.

According to an aspect of the present disclosure, the determining the three-dimensional candidate points may include generating nodes corresponding to the three-dimensional candidate points for each depth according to an arrangement order of the reference points along the center line of the segmented blood vessel region in the reference image, and connecting the generated nodes through an edge.

According to an aspect of the present disclosure, the connecting the generated nodes through the edge may include, in response to a distance between the three-dimensional candidate points corresponding to nodes of adjacent depths of the generated nodes being within a threshold distance, connecting the nodes of the adjacent depths.

According to an aspect of the present disclosure, the connecting the generated nodes through the edge may include determining an edge weight for the edge based on a distance between three-dimensional candidate points corresponding to two nodes connected to the edge.

According to an aspect of the present disclosure, the connecting the generated nodes through the edge may include determining an edge weight for the edge based on a distance between an epipolar line to a reference point and an epipolar line to a sub-point corresponding to a three-dimensional candidate point of any one of the two nodes connected to the edge.

According to an aspect of the present disclosure, the determining the three-dimensional path may include determining the three-dimensional path by selecting waypoints from among the three-dimensional candidate points between a start point and an end point of the center line of the reference image.

According to an aspect of the present disclosure, the determining the three-dimensional path may include determining the three-dimensional path based on edge weights of nodes corresponding to the three-dimensional candidate points in the tree structure data.

According to an aspect of the present disclosure, the determining the three-dimensional path may include determining the three-dimensional path from potential paths based on the three-dimensional candidate points, based on a cost calculated using an edge weight of the tree structure data.

According to an aspect of the present disclosure, the determining the three-dimensional diameter for each of the points on the three-dimensional path may include identifying two-dimensional points corresponding to points on the three-dimensional path in the reference image and the sub-image, and calculating the three-dimensional diameter based on at least one of widths of the identified two-dimensional points.

According to an aspect of the present disclosure, the calculating the three-dimensional diameter may include calculating an average value of the widths of the identified two-dimensional points as the three-dimensional diameter.

According to an aspect of the present disclosure, the reconstructing the three-dimensional shape of the blood vessels may include generating vertices along a circumference corresponding to the three-dimensional diameter at each of the points on the three-dimensional path, and reconstructing the three-dimensional shape of the blood vessels using a mesh based on the generated vertices.

There is provided a non-transitory computer-readable recording medium storing instructions for executing the method to reconstruct a three-dimensional shape of blood vessels on a computer.

According to an aspect of the present disclosure, an electronic device may comprise an image acquisition unit configured to acquire medical images captured at a plurality of positions, and a processor configured to generate tree structure data of three-dimensional candidate points corresponding to points along center lines of blood vessel regions of a reference image and a sub-image of the medical images, determine a three-dimensional path based on the tree structure data, determine a three-dimensional diameter for each of the points on the three-dimensional path, and reconstruct the three-dimensional shape of the blood vessels corresponding to the three-dimensional path based on the determined three-dimensional diameter.

With the reconstruction of the 3D shape of the blood vessels according to some aspects, it is possible to accurately reconstruct the 3D shape of the blood vessels even for a position of severe bent in the blood vessels.

With the reconstruction of the 3D shape of the blood vessels according to some aspects, it is possible to reconstruct the 3D shape of the blood vessels more quickly.

With the reconstruction of the 3D shape of the blood vessels according to some aspects, it is possible to accurately reconstruct the 3D shape of the blood vessels even for a section in which the epipolar line and the center line of the blood vessels are parallel.

## BRIEF DESCRIPTION OF THE DRAWINGS

The above and other objects, features and advantages of the present disclosure will be described with reference to the accompanying drawings described below, where similar reference numerals indicate similar elements, but not limited thereto, in which:

FIG. 1 is a diagram illustrating a structure of a medical electronic device;
FIG. 2 is a diagram provided to explain a change in the projection angle of radiation according to the rotation of the C-arm included in the electronic device;
FIG. 3 is a diagram provided to explain a process of calculating a linear distance from the radiation source to the target blood vessels by the electronic device;
FIG. 4 is a flowchart illustrating a method to reconstruct a 3D shape of blood vessels;
FIGS. 5 to 7 illustrate generation of 3D candidate points;
FIG. 8 illustrates filtering of the 3D candidate points according to some aspects;
FIGS. 9 to 11 illustrate generation of tree structure data according to some aspects;
FIG. 12 illustrates edge weights in the tree structure data according to some aspects;
FIG. 13 illustrates an example of a 3D path selected using the edge weights of the tree structure data according to some aspects;
FIGS. 14 and 15 are provided to explain matching between 3D points and 2D points by pair matching according to some aspects; and

FIG. 16 illustrates reconstruction of a 3D shape according to some aspects.

## DETAILED DESCRIPTION

Hereinafter, example details for the practice of the present disclosure will be described in detail with reference to the accompanying drawings. However, in the following description, detailed descriptions of well-known functions or configurations will be omitted if it may make the subject matter of the present disclosure rather unclear.

In the accompanying drawings, the same or corresponding components are assigned the same reference numerals. In addition, in the following description of various examples, duplicate descriptions of the same or corresponding components may be omitted. However, even if descriptions of components are omitted, it is not intended that such components are not included in any example.

Advantages and features of the disclosed examples and methods of accomplishing the same will be apparent by referring to examples described below in connection with the accompanying drawings. However, the present disclosure is not limited to the examples disclosed below, and may be implemented in various forms different from each other, and the examples are merely provided to make the present disclosure complete, and to fully disclose the scope of the disclosure to those skilled in the art to which the present disclosure pertains.

The terms used herein will be briefly described prior to describing the disclosed example(s) in detail. The terms used herein have been selected as general terms which are widely used at present in consideration of the functions of the present disclosure, and this may be altered according to the intent of an operator skilled in the art, related practice, or introduction of new technology. In addition, in specific cases, certain terms may be arbitrarily selected by the applicant, and the meaning of the terms will be described in detail in a corresponding description of the example(s). Accordingly, the terms used in this disclosure should be defined based on the meaning of the term and the overall content of the present disclosure, rather than simply the name of the term.

As used herein, the singular forms "a," "an," and "the" are intended to include the plural forms as well, unless the context clearly indicates the singular forms. Further, the plural forms are intended to include the singular forms as well, unless the context clearly indicates the plural forms. Further, throughout the description, when a portion is stated as "comprising (including)" a component, it is intended as meaning that the portion may additionally comprise (or include or have) another component, rather than excluding the same, unless specified to the contrary.

Further, the term "module" or "unit" used herein refers to a software or hardware component, and "module" or "unit" performs certain roles. However, the meaning of the "module" or "unit" is not limited to software or hardware. The "module" or "unit" may be configured to be in an addressable storage medium or configured to play one or more processors. Accordingly, as an example, the "module" or "unit" may include components such as software components, object-oriented software components, class components, and task components, and at least one of processes, functions, attributes, procedures, subroutines, program code segments, drivers, firmware, micro-codes, circuits, data, database, data structures, tables, arrays, and variables. Furthermore, functions provided in the components and the "modules" or "units" may be combined into a smaller number of components and "modules" or "units", or further divided into additional components and "modules" or "units."

A "module" or "unit" may be implemented as a processor and a memory, or may be implemented as a circuit (circuitry). Terms such as circuit and circuitry may refer to circuits in hardware, but may also refer to circuits in software. The "processor" should be interpreted broadly to encompass a general-purpose processor, a central processing unit (CPU), a microprocessor, a digital signal processor (DSP), a neural processing unit (NPU), a controller, a microcontroller, a state machine, etc. Under some circumstances, the "processor" may refer to an application-specific integrated circuit (ASIC), a programmable logic device (PLD), a field-programmable gate array (FPGA), etc. The "processor" may refer to a combination for processing devices, e.g., a combination of a DSP and a microprocessor, a combination of a plurality of microprocessors, a combination of one or more microprocessors in conjunction with a DSP core, or any other combination of such configurations. In addition, the "memory" should be interpreted broadly to encompass any electronic component that is capable of storing electronic information. The "memory" may refer to various types of processor-readable media such as random access memory (RAM), read-only memory (ROM), non-volatile random access memory (NVRAM), programmable read-only memory (PROM), erasable programmable read-only memory (EPROM), electrically erasable PROM (EEPROM), flash memory, magnetic or optical data storage, registers, etc. The memory is said to be in electronic communication with a processor if the processor can read information from and/or write information to the memory. The memory integrated with the processor is in electronic communication with the processor.

In addition, terms such as first, second, A, B, (a), (b), etc. used in the following examples are only used to distinguish certain components from other components, and the nature, sequence, order, etc. of the components are not limited by the terms.

In addition, in the following examples, if a certain component is stated as being "connected," "combined" or "coupled" to another component, it is to be understood that there may be yet another intervening component "connected," "combined" or "coupled" between the two components, although the two components may also be directly connected or coupled to each other.

In addition, as used in the following examples, "comprise" and/or "comprising" does not foreclose the presence or addition of one or more other elements, steps, operations, and/or devices in addition to the recited elements, steps, operations, or devices.

Hereinafter, various examples of the present disclosure will be described in detail with reference to the accompanying drawings.

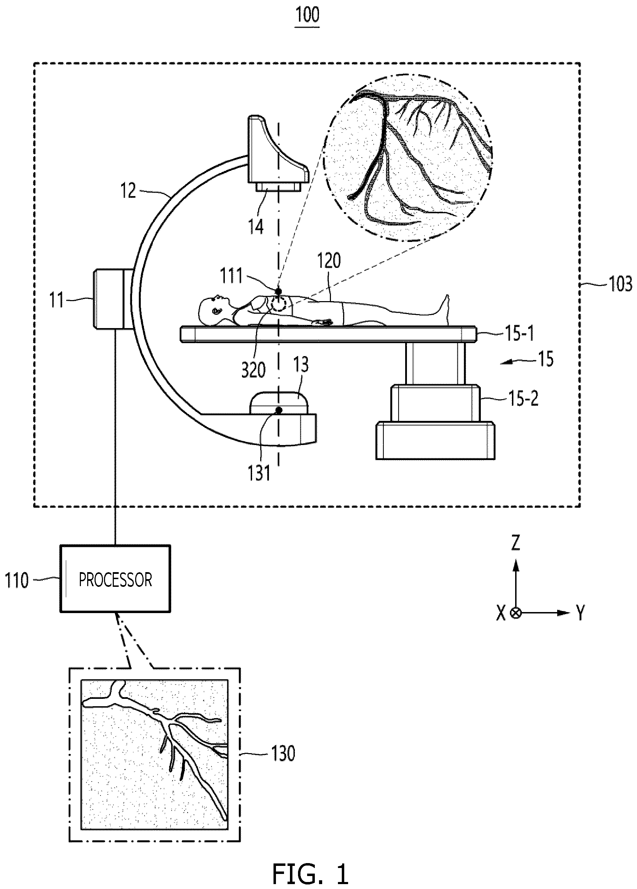

FIG. 1 is a diagram illustrating a structure of a medical electronic device.

In some aspects, a medical electronic device 100 (hereinafter, 'electronic device') may reconstruct a three-dimensional (3D) shape of a target from a medical image. The medical image of blood vessels of a subject of treatment may also be referred to as a blood vessels image. The electronic device 100 may reconstruct a 3D shape of the blood vessels, and may also be referred to as a 3D blood vessel shape reconstruction device. The 3D shape of the blood vessels reconstructed by the electronic device 100 may be used for calculating indicators related to blood flow (e.g., Quantitative Flow Ratio (QFR), Fractional Flow Reserve (FFR)).

The electronic device 100 may include an image acquisition unit and a processor 110. Although the image acquisition unit as an imaging device will be mainly described herein by way of example, aspects are not limited thereto, and the image acquisition unit may be a device that receives a medical image (e.g., a blood vessel image) from an external imaging device based on wired and/or wireless communication.

The image acquisition unit may include a device that irradiates radiation to the blood vessels of a subject of treatment and captures blood vessel images. Examples of the types of blood vessels may include Left Main Coronary Artery (LM), Left Anterior Descending Artery (LAD), Left Circumflex Artery (LCX), Right Coronary Artery (RCA), etc. The image acquisition unit may capture the blood vessel images using coronary angiography. For example, the image acquisition unit may include a body unit 11, a C-arm 12, a radiation irradiation unit 13, and a radiation detection unit 14. An imaging device having the C-arm 12 may also be referred to as a C-arm imaging device.

The C-arm 12 may have a curved C-shaped arc shape with one side open. For example, if the C-arm 12 is erected vertically with respect to a floor on which the electronic device 100 is placed, the C-arm 12 may have a symmetrical shape with respect to a plane including an isocenter 111 and parallel to the floor. The isocenter 111 is a portion or point as a center of the radiation beams emitted from various positions despite the rotation of the C-arm, and may be defined as a point at which a first rotation axis and a second rotation axis of the C-arm intersect each other. The isocenter 111 may represent a center of a rotation trajectory of the radiation irradiation unit 13 (or radiation source (not shown)) generated according to the rotation of the C-arm 12. The C-arm 12 may have a shape that is open toward the isocenter 111.

The body unit 11 may be connected to the C-arm 12. The body unit 11 may be mechanically coupled to the C-arm 12. The C-arm 12 may be rotated with respect to the body unit 11. The C-arm 12 may be rotated in a YZ plane with respect to the body unit 11. For example, the protrusion included in the body unit 11 and a moving guide included in the C-arm 12 may be coupled with each other, and the C-arm 12 may rotate in the YZ plane along an rotation axis parallel to an X axis and passing through the isocenter 111. In addition, the C-arm 12 may be rotated in an XZ plane with respect to the body unit 11. For example, with a point at which the body unit 11 and the C-arm 12 meet being in a fixed state, the C-arm 12 may rotate in the XZ plane with respect to a rotation axis parallel to the Y axis and passing through the isocenter 111.

The radiation irradiation unit 13 and the radiation detection unit 14 may be disposed on an inner surface of the C-arm 12, while facing each other with the isocenter 111 interposed therebetween. Each of the radiation irradiation unit 13 and the radiation detection unit 14 may be connected to the C-arm 12. The radiation irradiation unit 13 may include one or more radiation sources and may emit radiation toward the subject of treatment through the one or more radiation sources. The radiation detection unit 14 may include a radiation detection sensor, and may detect, through the radiation detection sensor, radiation that is emitted from the radiation irradiation unit 13 and transmitted through the blood vessels of the subject of treatment. Since the radiation irradiation unit 13 and the radiation detection unit 14 are connected through the C-arm 12 and rotated integrally, if the radiation detection unit 14 is moved, the radiation irradiation unit 13 may be located on the opposite side of the radiation detection unit 14 based on the isocenter 111. As will be described below, the electronic device 100 may capture medical images (e.g., blood vessel images) at a plurality of image capturing positions while changing the position (e.g., the image capturing position) of the radiation detection unit 14.

The subject of treatment may be laid on a table 15. More specifically, the table 15 may include a table top 15-1 on which the subject of treatment can be laid, and a table support 15-2 supporting the table top 15-1. The table support 15-2 may be fixed to the floor. For example, the table top 15-1 may be movably coupled with respect to the table support 15-2. The electronic device 100 may further include an actuator (not shown) that changes a position of the table top 15-1 with respect to the table support 15-2. The electronic device 100 may move the table top 15-1 with respect to the table support 15-2 through the actuator (not shown). The actuator (not shown) may include a motor (e.g., an electric motor) and a power transmission structure.

The table top 15-1 may move in a left-right direction and an up-down direction with respect to the table support 15-2. For example, the electronic device 100 may move the table top 15-1 in the left-right direction on a plane (e.g., an XY plane) parallel to a surface of the table. The surface of the table may represent an upper surface of the table top 15-1. The electronic device 100 may move the table top 15-1 in the up-down direction on an axis (e.g., a Z-axis) perpendicular to the plane (e.g., the XY plane) parallel to the surface of the table. In the following description, moving the table 15 may refer to moving the table top 15-1 with respect to the table support 15-2. For example, the electronic device 100 may move the table 15 so as to accurately irradiate radiation to the target blood vessels of the subject of treatment.

The electronic device 100 may control the C-arm 12 to rotate around the subject of treatment located on the table 15. After fixing the C-arm 12, the electronic device 100 may irradiate the target blood vessels of the subject of treatment on the table 15 through the radiation source of the radiation irradiation unit 13 connected to the C-arm 12. The radiation irradiated to the blood vessels of the subject of treatment may be X-rays.

In some aspects, the subject 120 of treatment may be positioned on the table top 15-1 of the electronic device 100. The electronic device 100 may generate a blood vessel image 130 of target blood vessels 320 of the subject 120 of treatment captured by using the radiation emitted from the radiation source 131.

The processor 110 of the electronic device 100 may derive a quantitative analysis result such as a diameter of the target blood vessels 320, a length of a lesion, etc. from the generated blood vessel image 130. The processor 110 may convert a distance measured in the blood vessel image 130 into a real-world physical distance such as $\mu$m, mm, cm, so as to derive a quantitative analysis result related to the target blood vessels 320 from the blood vessel image 130. For example, the processor 110 may utilize a calibration factor to convert the distance measured in the vascular image 130 into the physical distance.

Specifically, the physical distance may be calculated by multiplying the calibration factor by the number of pixels corresponding to the distance measured in the blood vessel image 130. For example, the physical distance may be expressed as Equation 1 below.

<Equation 1>

$$Physical\ distance = Calibration\ factor\ x\ Number\ of\ pixels$$

Referring to Equation 1, the calibration factor may represent a physical distance corresponding to the length (e.g., horizontal length or vertical length) of one of pixels forming the vascular image 130. In order to accurately calculate the physical distance, it is necessary to accurately calculate the calibration factor.

The calibration factor may represent a spatial relationship between the object in the blood vessel image and the object in the real world. The calibration factor may be calculated by multiplying a quotient obtained by dividing a source to object distance (SOD) by a source to image receptor distance (SID), by an imager pixel spacing. The imager pixel spacing may represent the length (e.g., horizontal length or vertical length) of one of the pixels forming the blood vessel image. That is, the calibration factor may be expressed as Equation 2 below.

<Equation 2>

$$Calibration\ factor = SOD/SID\ x\ Imager\ pixel\ spacing$$

where, SOD may represent a linear distance from the source (e.g., the radiation source) to the object for imaging, and SID may represent a linear distance from the source to the image receptor. The SID and the imager pixel spacing may be extracted from metadata of Digital Imaging and Communications in Medicine (DICOM). The DICOM may represent a standard for the storage and transmission of data related to images generated in a medical electronic device.

In some aspects, the electronic device 100 may irradiate the target blood vessels 320 of the subject 120 of treatment positioned on the table top 15-1 by using the radiation source 131 included in the radiation irradiation unit 13. The electronic device 100 may calculate a target distance from the radiation source 131 to the target blood vessels 320 based on a first vertical distance from the isocenter 111 to the table 15 and a second vertical distance from the target blood vessels 320 to the table 15. The first vertical distance from the isocenter 111 to the table 15 may represent a vertical distance from the isocenter 111 to the upper surface of the table top 15-1. Likewise, the second vertical distance from the target blood vessels 320 to the table 15 may represent a vertical distance from the target blood vessels 320 to the upper surface of the table top 15-1.

More specifically, the electronic device 100 may adjust the position of the target blood vessels 320 through the table 15 before irradiating the target blood vessels 320 with radiation using the radiation source 131. The electronic device 100 may move the table 15 so that the radiation source 131, the target blood vessels 320, and the isocenter 111 are disposed on a straight line. In some aspects, the electronic device 100 may move the table top 15-1 only in the left-right direction on the XY plane so that the radiation source 131, the target blood vessels 320, and the isocenter 111 are disposed on a straight line. In other aspects, the electronic device 100 may move the table top 15-1 in both the left-right direction on the XY plane and the up-down direction on the Z-axis so that the radiation source 131, the target blood vessels 320, and the isocenter 111 are disposed on a straight line.

In some aspects, if the position of the radiation source 131 changes according to the rotation of the C-arm 12, the table top 15-1 may be moved together so that the radiation source 131, the target blood vessels 320, and the isocenter 111 are disposed on a straight line.

In some aspects, the electronic device 100 may move the table 15 in the left-right direction on a plane including the surface of the table so that the isocenter 111 is disposed on the same straight line as the radiation source 131 and the target blood vessels 320. A height of the table 15 may be fixed, but aspects are not limited thereto, and the height may be varied. For example, the electronic device 100 may calculate a height of the target blood vessels 320 from the floor. The electronic device 100 may calculate the height of the target blood vessels 320 from the floor by summing the height of the target blood vessels 320 from the surface of the table and the height of the table 15 from the floor. However, aspects are not limited thereto, and the electronic device 100 may acquire a height from the table 15 (e.g., from the surface of the table) to the target blood vessels 320. The electronic device 100 may generate a plane including a point corresponding to the target blood vessels 320 while parallel to the floor (or XY plane), based on the position (e.g., height) of the target blood vessels 320. The electronic device 100 may calculate a plane parallel to the floor and including the target blood vessels 320, and a point ('intersection point') intersecting a straight axis connecting the radiation source 131 and the isocenter 111. The electronic device may move the table top 15-1 in the left-right direction on the XY plane so that the target blood vessels 320 is located at the calculated intersection point.

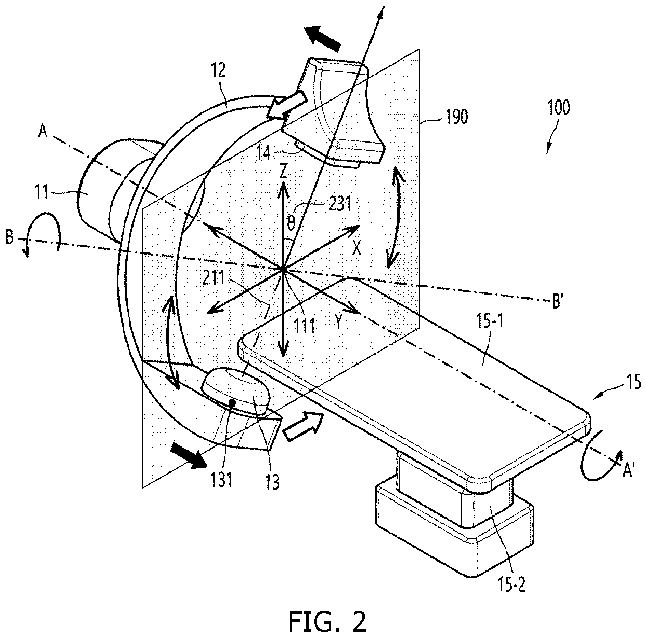

In some aspects, after adjusting the position of the target blood vessels 320 through the movement of the table top 15-1, the electronic device 100 may calculate a target distance representing a linear distance from the radiation source 131 to the target blood vessels 320 using a plurality of parameters. The plurality of parameters may include, for example, a position of the table 15, a position of the target blood vessels 320, and an irradiation angle of the radiation emitted from the radiation source 131 (e.g., a projection angle 231 of the radiation of FIG. 2). More specifically, the electronic device 100 may accurately calculate the target distance from the radiation source 131 to the target blood vessels 320 based on the first vertical distance from the isocenter 111 to the table 15, the second vertical distance from the target blood vessels 320 to the table 15, and the irradiation angle of radiation emitted from the radiation source 131.

FIG. 2 is a diagram provided to explain a change in the projection angle of radiation according to the rotation of the C-arm included in the electronic device.

In some aspects, the electronic device 100 may irradiate the target blood vessels of the subject of treatment positioned on the table 15 by using the radiation source 131 included in the radiation irradiation unit 13.

In some aspects, the electronic device 100 may rotate the radiation source based on at least one of a first rotation axis A-A' including the isocenter 111 and parallel to the table plane and a second rotation axis B-B' different from the first rotation axis A-A'. For example, the radiation source may be rotated on one of a plane perpendicular to the first rotation axis A-A' including the isocenter 111 and parallel to the table plane, or a plane perpendicular to the second rotation axis B-B' including the isocenter 111 and different from the first rotation axis A-A'. The second rotation axis B-B' may intersect the first rotation axis A-A', and for example, they may be orthogonal to each other at the isocenter 111. For reference, an example in which the C-arm and radiation source are rotated on one plane is described for convenience of explanation, but aspects are not limited thereto, and they may be rotated based on two rotation axes A-A' and B-B' at the same time.

For example, the electronic device 100 may rotate the C-arm 12 with respect to the body unit 11 around the first rotation axis A-A'. The first rotation axis A-A' may represent an axis connecting the isocenter 111 and the point at which the C-arm 12 and the body unit 11 are coupled. The position of the radiation source 131 may change according to the rotation of the C-arm 12. For example, according to the rotation of the C-arm 12 around the first rotation axis A-A', the radiation source 131 may be rotated along a rotation trajectory on a plane 190 including the isocenter 111 and perpendicular to the first rotation axis A-A'. In FIG. 2, an X-Z plane is illustrated as an example of the plane 190 perpendicular to the first rotation axis A-A'.

The radiation source 131 may be rotated according to the rotation of the C-arm 12, along a rotation trajectory in a shape similar to a circle on the plane 190. When the C-arm 12 is rotated, the rotational trajectory of the radiation source 131 in motion may not be geometrically a perfect circle, and may be a partially squashed circular shape due to sagging, vibration, etc. of the C-arm 12.

In addition, the electronic device 100 may rotate the C-arm 12 with respect to the body unit 11 around the second rotation axis B-B'. The second rotation axis B-B' may be an axis passing through the isocenter 111 and intersecting (e.g., orthogonal to) the first rotation axis A-A'. For example, according to the rotation of the C-arm 12 around the second rotation axis B-B', the radiation source 131 may be rotated along a rotation trajectory on a plane including the isocenter 111 and perpendicular to the second rotation axis B-B'. For reference, FIG. 2 illustrates an example in which the second rotation axis B-B' is rotated together according to the rotation of the C-arm based on the first rotation axis A-A'. However, if the C-arm 12 is rotated based on the second rotation axis B-B' while the radiation source 131 and the radiation detection unit 14 are positioned on a Z axis, the second rotation axis B-B' is parallel to the X axis and the radiation source 131 may be rotated along a rotation trajectory on the Y-Z plane.

If the position of the radiation source 131 is changed according to the rotation of the C-arm 12, the projection angle 231 (e.g., θ) of the radiation emitted from the radiation source 131 may be changed. In other words, the projection angle 231 of the radiation emitted from the radiation source may be changed according to the rotation of the C-arm 12.

The projection angle 231 of radiation may represent an angle between an axis (e.g., the Z axis) orthogonal to the plane including the surface of the table 15 and an axis 211 corresponding to an irradiation direction of the radiation emitted from the radiation source 131. The irradiation direction of radiation may represent a direction from the radiation irradiation unit 13 (or the radiation source 131) toward the radiation detection unit 14 (or the radiation detection sensor). The axis 211 corresponding to the irradiation direction of radiation may represent an axis connecting the radiation irradiation unit 13 and the radiation detection unit 14. The projection angle 231 of the radiation may be -180 to 180 degrees.

The isocenter 111 may be variously defined based on the rotation of the C-arm 12. For example, the isocenter 111 may represent a point where the radiations irradiated from various positions of the radiation source according to the rotation of the C-arm 12 are densely concentrated. As another example, the isocenter 111 may represent the geometric center of the rotational trajectory of the radiation source 131. As another example, the isocenter 111 may represent a center of rotation of the C-arm 12.

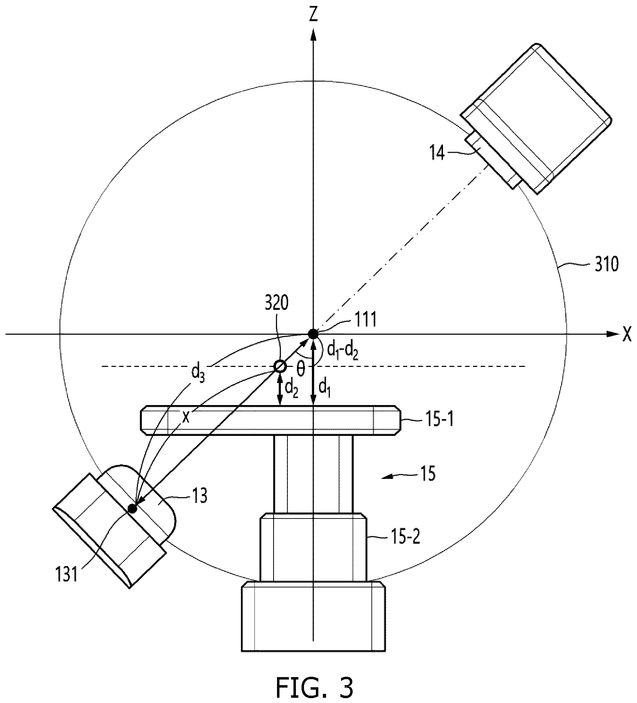

FIG. 3 is a diagram provided to explain a process of calculating a linear distance from the radiation source to the target blood vessels by the electronic device.

FIG. 3 is a side view of the electronic device (e.g., the electronic device 100 of FIG. 1) viewed in the XZ plane. The electronic device may move the table top 15-1 to align the radiation source 131, the target blood vessels 320, and the isocenter 111 on a straight line before irradiating the radiation to the target blood vessels 320 using the radiation source 131. As described above, the position (e.g., the image capturing position) of the radiation detector 14 may be moved according to the rotation of the C-arm 12. For example, the radiation detection unit 14 and the radiation irradiation unit 13 may be moved in an area range 310 corresponding to a surface of a hemisphere according to the rotation of the C-arm 12.

In some aspects, the electronic device may calculate a first value $(d_1 - d_2)$ by subtracting the second vertical distance $(d_2)$ from the first vertical distance $(d_i)$, in which the second vertical distance $(d_2)$ is a distance from the subject 120 of treatment (e.g., the target blood vessels) to the table 15 and the first vertical distance $(d_1)$ is a distance from the isocenter 111 to the table 15. The first value $(d_1 - d_2)$ may represent a vertical distance from the isocenter 111 to the target blood vessels 320. In addition, the electronic device may calculate a second value by dividing the calculated first value $(d_1 - d_2)$ by a cosine value $(\cos(è))$ of the irradiation angle of radiation (e.g., the projection angle of radiation of FIG. 2). The second value may represent a linear distance from the isocenter 111 to the target blood vessels 320. The electronic device may calculate a target distance (x) from the radiation source 131 to the target blood vessels 320 by subtracting the calculated second value (i.e., the linear distance from the isocenter 111 to the target blood vessels 320) from a linear distance $(d_3)$ from the radiation source 131 to the isocenter 111. For example, the target distance from the radiation source 131 to the target blood vessels 320 may be calculated as expressed in Equation 3 below.

<Equation 3>

$$SOD = Source\ to\ Isocenter - \frac{Isocenter\ to\ table - Object\ height}{\cos \theta}$$

where, *SOD* may represent a linear distance from the radiation source 131 to the target blood vessels 320, *Source to Isocenter* may represent a linear distance $(d_3)$ from the radiation source 131 to the isocenter 111, *Isocenter to table* may represent a vertical distance $(d_1)$ from the target blood vessels 320 to the table 15, and θ may represent the irradiation angle of radiation.

In some aspects, after calculating the target distance, the electronic device may calculate a calibration factor based on the calculated target distance. As described above, the electronic device may utilize the calculated calibration factor to convert the distance in the blood vessel image 130 into a physical distance. As will be described below, in the present description, the electronic device may calculate the physical distance corresponding to the diameter of the target blood vessels by multiplying the number of pixels corresponding to the diameter of the target blood vessels appearing in the captured blood vessel image by the calibration factor.

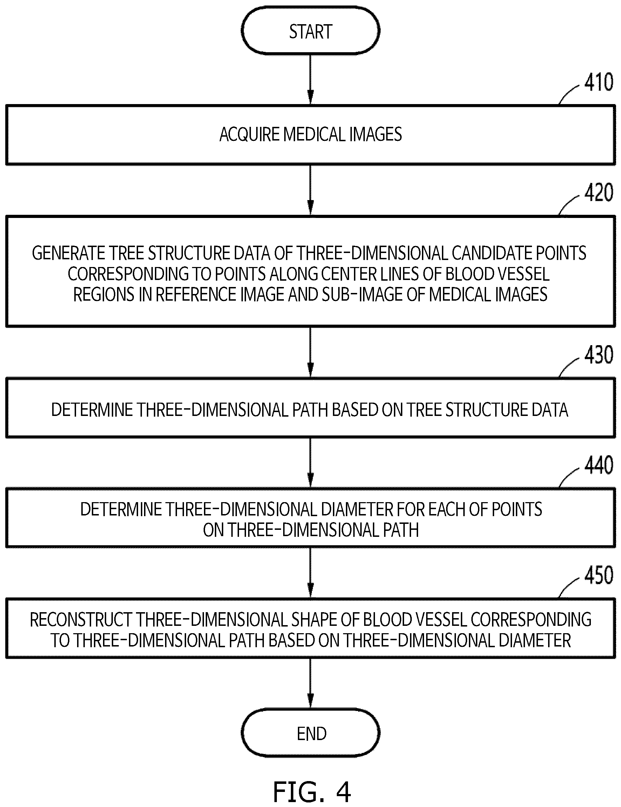

FIG. 4 is a flowchart illustrating a method to reconstruct a 3D shape of blood vessels.

At operation 410, the electronic device (e.g., the electronic device 100 of FIG. 1) may acquire medical images captured at a plurality of positions. For example, the electronic device may acquire the medical images from the plurality of image capturing positions based on the C-arm imaging device. For example, the electronic device may capture the medical images at each of the plurality of image capturing positions by moving the radiation irradiation unit and radiation detection unit of the C-arm imaging device through the rotation of the C-arm. For example, the electronic device may capture a plurality of medical images using the C-arm imaging device.

The electronic device may determine an image capturing parameter of a plurality of captured medical images. The image capturing parameter may refer to a parameter indicating the image capturing position and/or posture for the medical image, and may be, for example, a parameter indicating the position of the radiation irradiation unit and/or the radiation detection unit that captured the medical images and/or the irradiation angle of the radiation. The image capturing parameter may include a position recorded in a DICOM file.

The electronic device may correct the image capturing parameter of each of the medical images. The electronic device may perform an offset-correction algorithm. If the medical image is an image captured using the C-arm imaging device, due to the characteristics of the C-arm, there may occur a difference between the actual position and the position recorded in the Digital Imaging and Communications in Medicine (DICOM) file due to noise from the rotation of the machine (e.g., the C-arm). For reference, image capturing information (e.g., the image capturing parameters) of the DICOM file may include an angle, a source to image detector distance (SID), and a table position. In order to calibrate the noise caused due to the characteristics of the C-arm, the electronic device may optimize the image capturing parameters through the image capturing information of the acquired DICOM files and Common-Image point (CIP) pairs. The Common-Image point (CIP) pairs may refer to a pairs of points representing a common position in medical images captured at different image capturing positions and/or irradiation angles and may include, for example, a proximal point representing a starting point of a blood vessel region and a distal point representing an end point of the blood vessel region. For example, the CIP pair may include a proximal point in a reference image and a proximal point in a sub-image. Additionally, the CIP pair may include a distal point in the reference image and a distal point in the sub-image. The electronic device may optimize an objective function (e.g., minimize an objective function value) by changing, through optimizers such as LM and SGD, the image capturing parameters of the second DICOM file, the SID of the first DICOM. For example, in the case of backprojection after creating 3D points through two-dimensional CIP pairs and image capturing parameters, the objective function may be a function that calculates the distance value to two-dimensional CIP pairs.

At operation 420, the electronic device may generate tree structure data of 3D candidate points corresponding to points along center lines of blood vessel regions of the reference and the sub-images of the medical images. For example, the electronic device may set one of two medical images as the reference image. The electronic device may determine another medical image to be the sub-image. The electronic device may segment the blood vessel region in each of the reference image and the sub-image. The electronic device may determine a center line with respect to the segmented blood vessel region of each of the images.

In the present description, the center line of the blood vessel region may refer to a line passing through the center of the blood vessel, and it may be a line connecting central points of the inner diameter of the blood vessel in a longitudinal direction of the blood vessel. The points along the center line may indicate points at positions spaced apart by a predetermined length along the center line from the starting position in the blood vessels region of the medical image (e.g., the blood vessel image). The diameter at the position along the center line may represent the length (or spacing) between inner walls of the blood vessel along a line perpendicular to the center line at a corresponding position, and may also be referred to as width. The center line of the blood vessel region in the reference image may be indicated as a reference center line, and the center line of the blood vessel region in the sub-image may be indicated as a sub-center line.

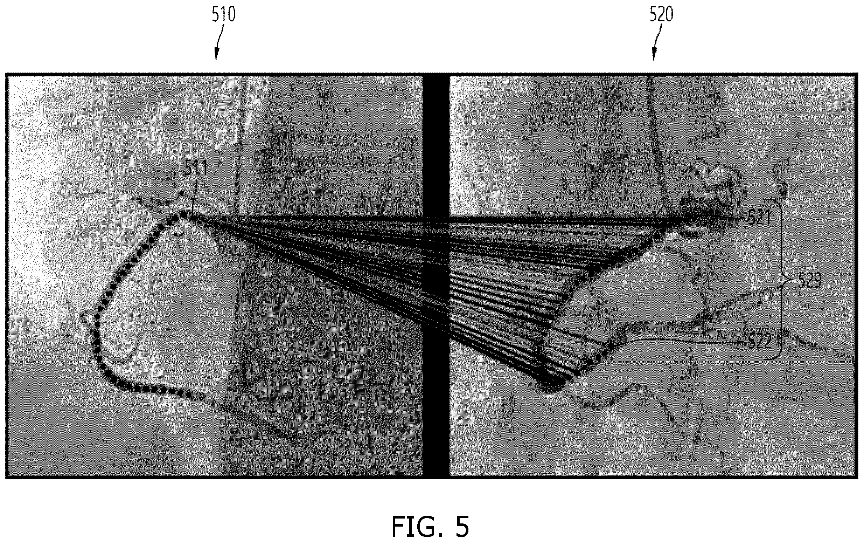

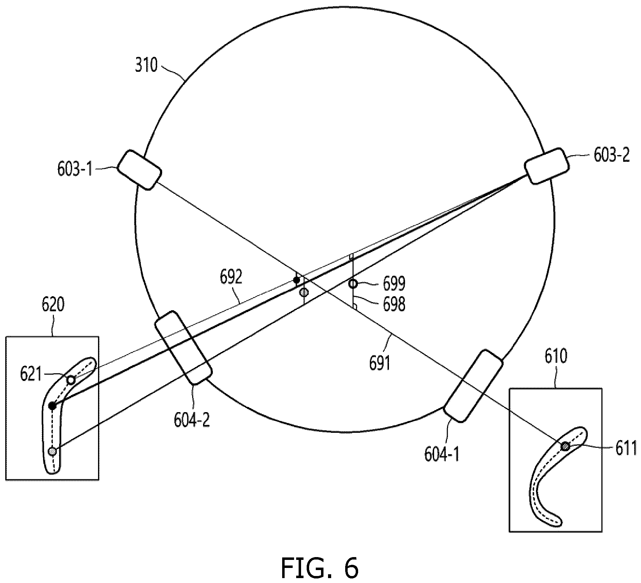

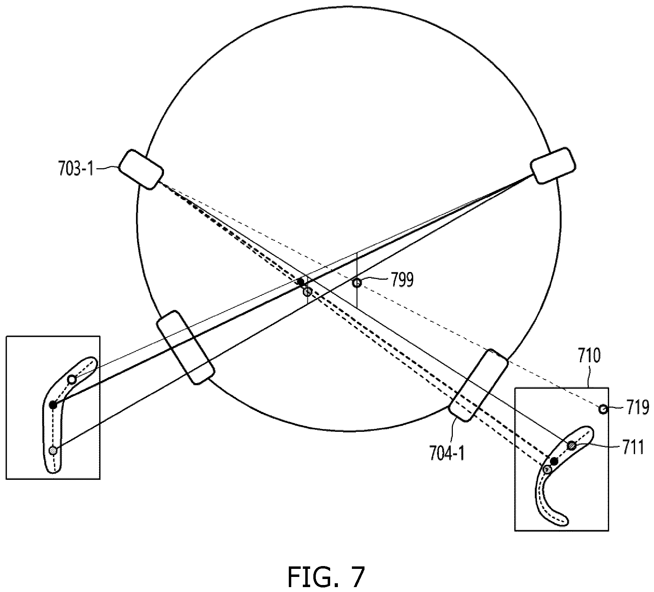

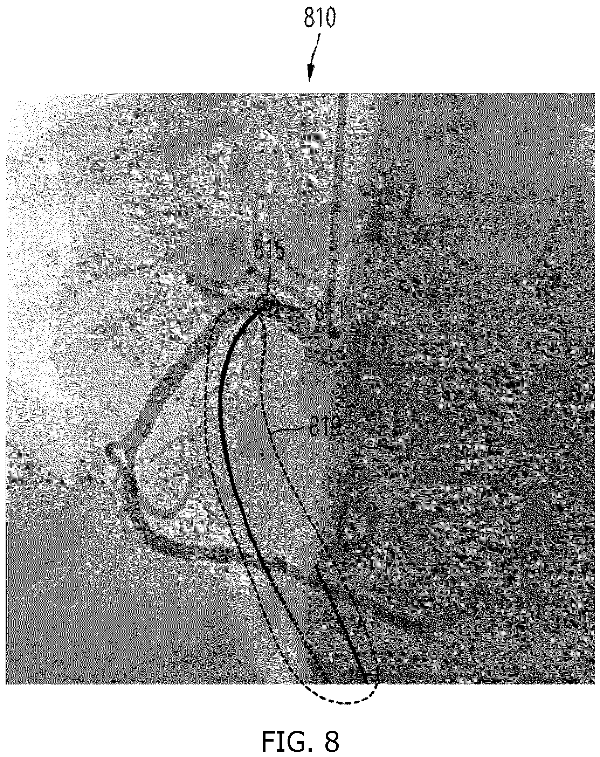

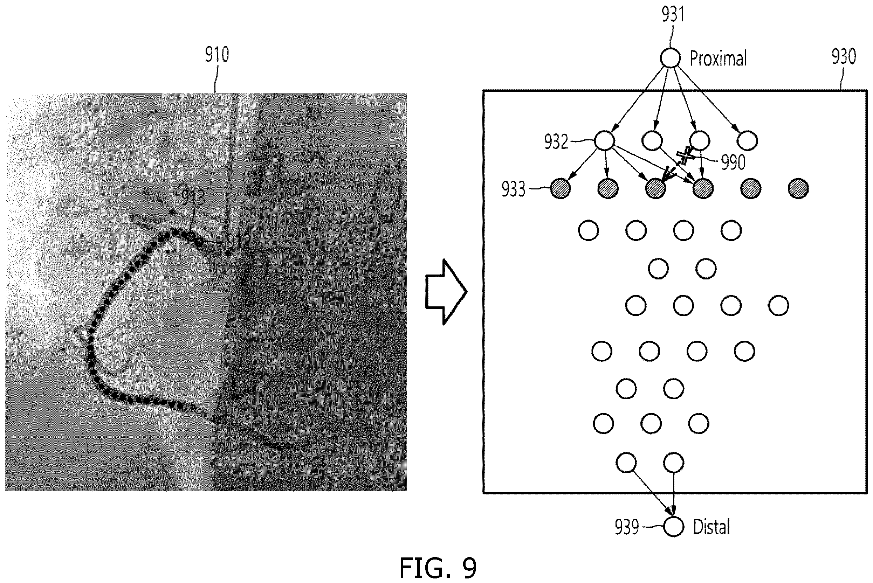

The electronic device may determine a 3D candidate point for each of the points (e.g., sub-points) of the sub-center line of the sub-image based on points (e.g., reference points) of the reference center line of the reference image. In the following description, FIG. 5 shows pairing a plurality of sub-points based on any reference point, FIG. 6 shows determining 3D candidate point for each of the pairs, FIG. 7 shows backprojecting the 3D candidate point as a reference image, and FIG. 8 shows filtering the 3D candidate point. FIG. 9 illustrates an example of generated tree structure data.

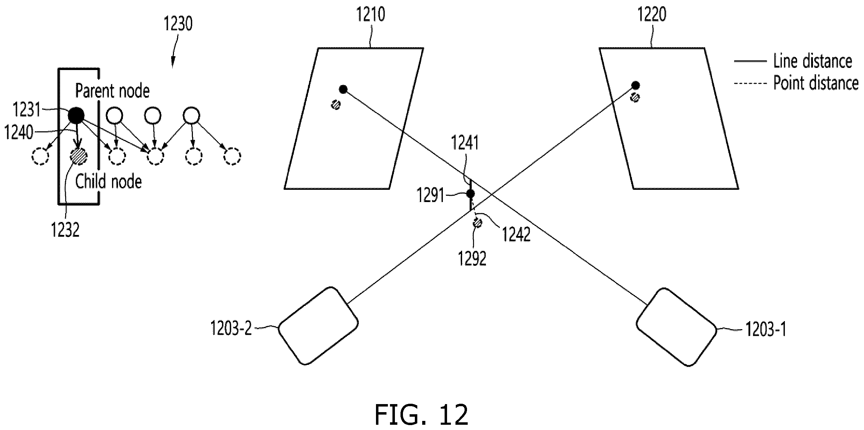

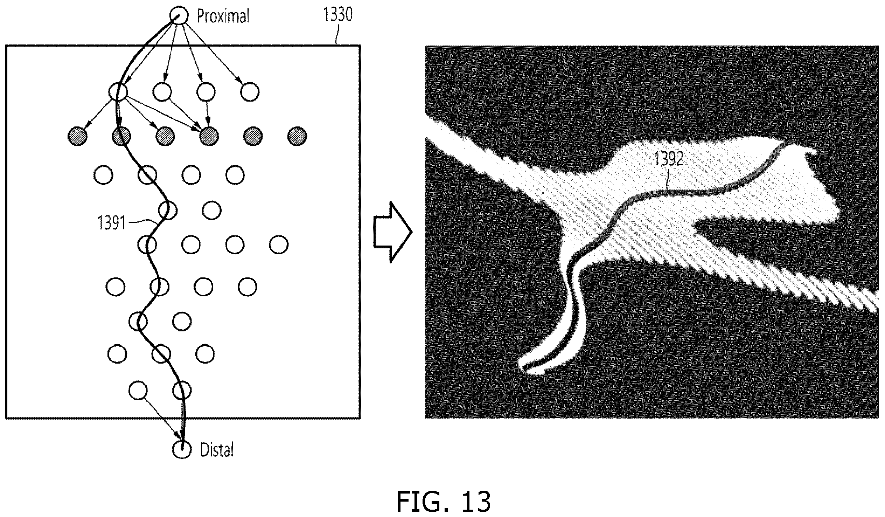

At operation 430, the electronic device may determine a 3D path based on the tree structure data. FIG. 12 illustrates an example of an edge weight of the tree structure data, and FIG. 13 illustrates an example of a 3D path determined using the tree structure data.

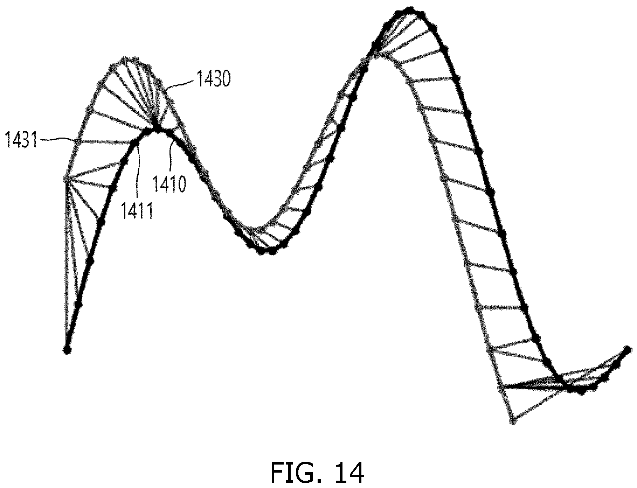

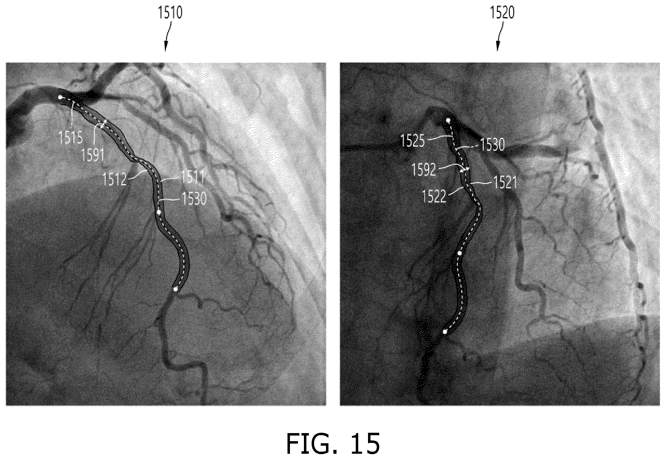

At operation 440, the electronic device may determine a 3D diameter for each point on the 3D path. FIGS. 14 and 15 illustrate an example of determining a 3D diameter using blood vessel widths calculated from a two-dimensional image, which will be described below.

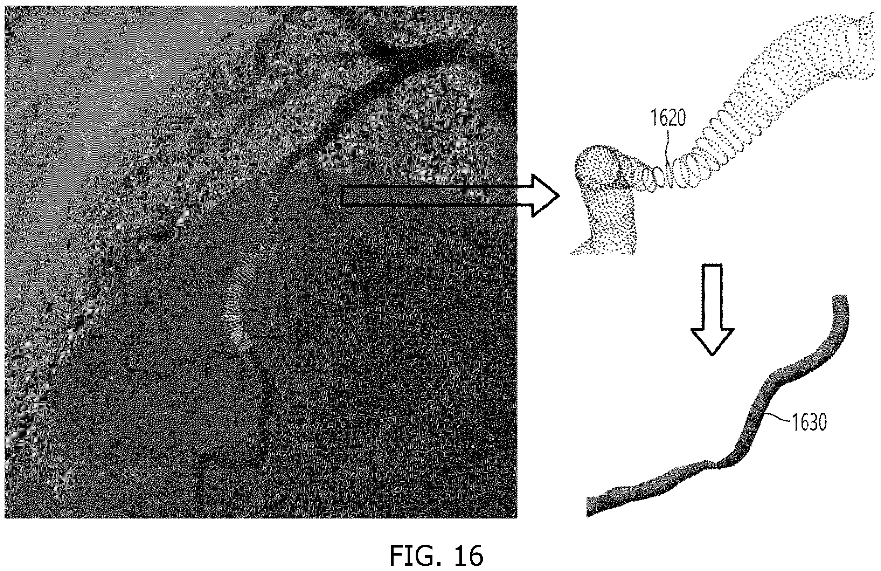

At operation 450, the electronic device may reconstruct the 3D shape of blood vessels corresponding to the 3D path based on the determined 3D diameter. An example of reconstructing a 3D shape of blood vessels will be described below with reference to FIG. 16.

FIGS. 5 to 7 illustrate generation of 3D candidate points.

In some aspects, the electronic device may segment a blood vessel region from the medical images (e.g., blood vessel images) captured as described above. The irradiation angle at which each medical image is captured may be different from the irradiation angle at which another medical image is captured. The electronic device may determine a center line (e.g., a blood vessel center line) with respect to the segmented blood vessel region of each medical image. In addition, the electronic device may acquire the CIP point and camera information in addition to the center line. The electronic device may generate a 3D center line through the center line obtained from the medical images (e.g., angiography images), the CIP point (e.g., proximal point, distal point) input from the user, and the camera information (e.g., camera parameters optimized through offset-calibration) at two image capturing positions. The 3D center line may include waypoints selected from the 3D candidate points (e.g., from a point cloud that is a set of 3D candidate points).

According to some aspects, the electronic device may determine a medical image, which is selected from among a plurality of medical images based on at least one of the length of the center line or lesion information, to be the reference image.

For example, the electronic device may select, as the reference image, a medical image having the shortest center line from among the plurality of medical images. For example, the electronic device may select an image having a shorter 2D center line among two medical images as the reference image. The electronic device may select an image having a longer 2D center line among two medical images as a sub-image. As another example, the electronic device may select a medical image from among the plurality of medical images based on the lesion information. The lesion information may refer to information on a lesion appearing in the medical image, and may include, for example, at least one of severity, size, position, or shape of the lesion. The severity of the lesion may refer to information on blood vessels constricted by the lesion, and may be, for example, percentage diameter stenosis (%DS). It may be expressed as %DS = 1 - (Diameter of lesion site)/(Normal diameter). The normal diameter is the diameter when it is assumed that the lesion site is normalized by the procedure, and for example, may be determined based on a statistical value (e.g., average value) of the diameter values of the normal site around the lesion site. The electronic device may identify lesion sites for each of a plurality of medical images and calculate lesion information for each of the identified lesion sites. For example, the electronic device may calculate %DS for each lesion in each medical image. When there are a plurality of lesions in each medical image, the electronic device may calculate a plurality of %DS values for each medical image. The electronic device may determine a maximum %DS value of a plurality of %DS values of each medical image. The electronic device may compare the maximum %DS values of the medical images. The electronic device may determine a medical image, which has the largest %DS value (e.g., maximum %DS value) among the plurality of medical images, to be the reference image. As another example, the electronic device may select the reference image in consideration of both the length of the center line and the lesion information described above.

The electronic device may determine 3D candidate points corresponding to the sub-points along the center line of the segmented blood vessel region in the reference image, for each of the reference points along the center line of the segmented blood vessel region. The electronic device may determine N points (e.g., reference points) along the center line (e.g., reference center line) in the reference image. The electronic device may determine M points (e.g., sub-points) along the center line (e.g., sub-center line) in the sub-image. N and M may be integers greater than or equal to 1. The electronic device may determine 3D points (e.g., 3D candidate points) for each of M sub-points for each of the N reference points.

Referring to FIG. 5, the electronic device may map one reference point 511 to M sub-points 529. For example, the electronic device may determine M number of 3D candidate points based on the assumption that they point to the same physical point (e.g., a physical point in a 3D space). The reference point 511 may be a pixel point in the reference image 510 which is a two-dimensional image, and the sub-point may be a pixel point in the sub-image 520 which is a two-dimensional image. Since the electronic device maps the M sub-points 529 to each of the N reference points, N×M mappings may be formed between the reference points and the sub-points 529.

Referring to FIG. 6, the electronic device may determine 3D candidate points for each of the mappings described above in FIG. 5. For example, based on the assumption that the reference point (e.g., the reference point 511 of FIG. 5) and the first sub-point (e.g., the first sub-point 521 of FIG. 5) point to the same physical point (e.g., 3D point), the electronic device may determine a 3D point corresponding to the mapping of the reference point (e.g., the reference point 511 of FIG. 5) and the first sub-point (e.g., the first sub-point 521). Similarly, based on the assumption that the reference point (e.g., the reference point 511 of FIG. 5) and the second sub-point (e.g., the second sub-point 522 of FIG. 5) point to the same physical point, the electronic device may determine a 3D point corresponding to the mapping of the reference point (e.g., the reference point 511 of FIG. 5) and the second sub-point (e.g., the second sub-point 522 of FIG. 5). FIG. 6 illustrates a brief example in which three sub-points are mapped to one reference point 611 for convenience of explanation, but 3D candidate points may be determined for the mappings of all M sub-points.

For reference, FIG. 6 is a more simplified side view of the electronic device (e.g., the electronic device 100 of FIG. 1) viewed from the XZ plane, in which radiation irradiation units 603-1 and 604-2 corresponding to the radiation irradiation unit 13 and the radiation detection unit 14 of FIG. 3 are shown. In an example in which the radiation irradiation units 603-1 and 603-2 and radiation detection units 604-1 and 604-2 are moved along a circular trajectory in the area range 310, the radiation detection unit 604-1 may capture images based on the radiation irradiated from the radiation irradiation unit 603-1 at the reference position, and the radiation detection unit 604-2 may capture images based on the radiation irradiated from the radiation irradiation unit 603-2 at the sub-position.

According to some aspects, the electronic device may determine 3D candidate points corresponding to the reference point 611 and sub-point 621 through triangulation. For example, the electronic device may determine a 3D candidate point based on a center point 699 on a line 698 orthogonal to a line 691 passing through the radiation irradiation unit 603-1 at the reference position that captured the reference image 610 and a point on the image plane corresponding to the corresponding reference point 611 of the reference image 610, and orthogonal to a line 692 passing through the radiation irradiation unit 603-2 at the sub position that captured the sub image 620 and a point on the image plane corresponding to the corresponding sub-point 621 of the sub image 620, or the intersection of the two lines. The image plane corresponding to the reference image 610 may refer to a plane corresponding to the radiation detection unit 604-1 at the reference position, and the image plane corresponding to the sub-image 620 may refer to a plane corresponding to the radiation detection unit 604-2 at the sub-position. The line passing through the radiation irradiation unit and the point on the image plane of each image may also be referred to as an epipolar line. When the epipolar line corresponding to the reference point and the epipolar line corresponding to the sub-point intersect, the intersecting point may be determined as a 3D candidate point, and if the epipolar lines do not intersect, the center point 699 on a line intersecting the two epipolar lines at points at which the distance between the two lines is minimum, may be determined as a 3D candidate point. The electronic device may generate a 3D candidate point for each of a plurality of sub-points corresponding to any reference point of a plurality of reference points by repeating the operation described above. M number of 3D candidate points may be generated for one reference point. The electronic device may generate N×M 3D candidate points. A set of 3D candidate points may be referred to as a point cloud.

Referring to FIG. 7, the electronic device may backproject a plurality of 3D candidate points corresponding to the same reference point 711 into the reference image 710. For example, the electronic device may determine points on a reference image plane (e.g., a plane corresponding to a radiation detection unit 704-1 at the reference position) at which the ray emitted from a radiation irradiation unit 703-1 at the reference position and passing through each of a plurality of 3D candidate points 799 reaches, as projected points 719 of the 3D candidate points.

FIG. 8 illustrates filtering of the 3D candidate points according to some aspects.

The electronic device may refine (or filter) the plurality of 3D candidate points based on their projected positions on a reference image 810, respectively. For example, the electronic device may maintain a 3D candidate point of the plurality of 3D candidate points, which corresponds to a projected point 815 around a corresponding reference point 811, and exclude the other 3D candidate points 819. The electronic device may select 3D candidate points having a projected point within a first threshold distance from the reference point.

Thus, when the generated 3D candidate point is projected as a reference image, the projected points 815 near the reference point used in the generation may be used as the candidate points (e.g., 3D candidate points) of the 3D center line.

FIGS. 9 to 11 illustrate generation of tree structure data according to some aspects.

The electronic device according to some aspects may generate nodes corresponding to the 3D candidate points for each depth according to an arrangement order of the reference points along the center line of the segmented blood vessel region in a reference image 910. For example, the electronic device may generate nodes corresponding to 3D candidate points, including a node 931 corresponding to the proximal point and a node 939 corresponding to the distal point. The electronic device may connect the generated nodes through an edge.

The electronic device may generate tree structure data 930 based on the filtered 3D candidate points. The tree structure data 930 may include a plurality of nodes, and each node may be connected through an edge. For example, each node of the tree structure data 930 may represent a corresponding 3D candidate point. The electronic device may generate the tree structure data 930 by generating the nodes corresponding to the filtered 3D candidate points and connecting the generated nodes through edges.

The electronic device may generate the tree structure data 930 including hierarchically built nodes by setting a depth for each node. The depth of the individual node may be set based on the order in which a plurality of reference points are arranged along the center line from a start point (e.g., proximal point) to an end point (e.g., distal point). Each of the reference points arranged along the reference center line in the reference image 910 may have an index (e.g., a center line index) according to the order they are arranged. The electronic device may determine a depth of a node indicating a corresponding reference point according to the center line index of the reference point. For example, a node indicating a 3D candidate point generated based on a first point (e.g., index = 0) on the reference center line may have a depth of zero. A node indicating a 3D candidate point generated based on a second point (e.g., index=1) on the reference center line may have a depth of 1. As described above in FIGS. 7 and 8, since a plurality of 3D candidate points are generated for the same reference point 912, a plurality of nodes 932 of the same depth (e.g., depth of 1) may be generated. Similarly, a plurality of nodes 933 corresponding to a plurality of candidate points may be generated for a reference point 913 of the next depth.

The electronic device may generate an edge forming a connection between nodes based on the depths set at the nodes. The electronic device may generate an edge between nodes corresponding to adjacent depths. Each edge may form a connection between a node of a previous depth (e.g., depth of 0) and a node of a next depth (e.g., depth of 1). The edge may have directionality, and may have a node of a previous depth as a start node and a node of a next depth as an end node.

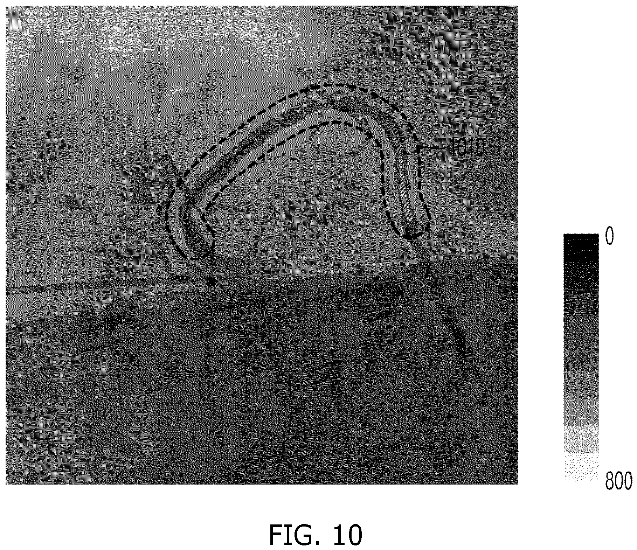

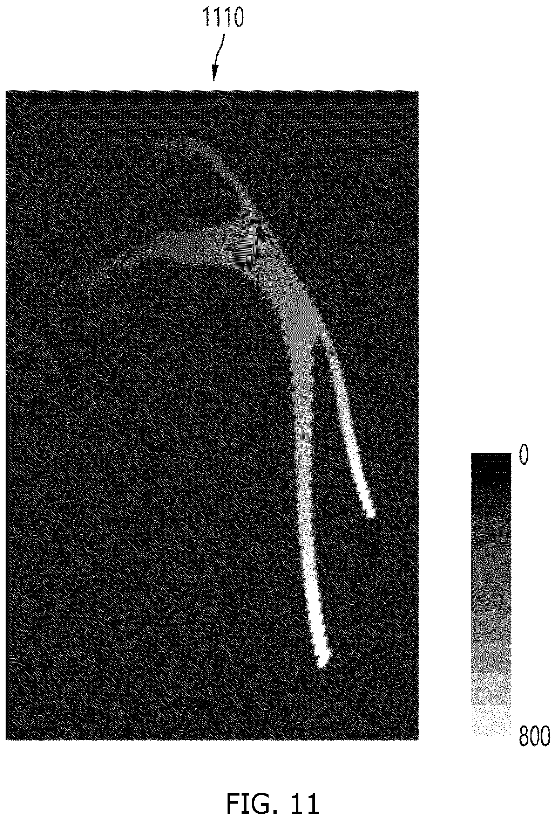

Additionally, in response to a distance between the 3D candidate points corresponding to the nodes of adjacent depths being within a threshold distance among the generated nodes, the electronic device may connect the nodes of adjacent depths. If the distance between a 3D candidate point indicated by the node of the first depth and a 3D candidate point indicated by the node of the second depth is within a second threshold distance, the electronic device may generate an edge between the node of the first depth and the node of the second depth. The first depth and the second depth may be adjacent to each other (e.g., they may be directly adjacent depths without an additional depth therebetween). If the distance between the 3D candidate point indicated by the node of the first depth and the 3D candidate point indicated by the node of the second depth exceeds the second threshold distance, the electronic device may exclude edge generation between the node of the first depth and the node of the second depth. For example, if the distance between a third point of the 3D candidate points corresponding to the first reference point and a fourth point of the 3D candidate points corresponding to the second reference point exceeds the second threshold distance, an edge 990 may not be connected between the two points. FIG. 10 illustrates an example in which 3D candidate points 1010 divided by the depths of the tree structure data are backprojected onto an image plane (e.g., a reference image plane). FIG. 11 is an arbitrary view showing a visualization 1110 of a point cloud including 3D candidate points of the tree structure data. Although FIGS. 10 and 11 show points starting from a point corresponding to the 0th index (e.g., depth of 0) and ending at the 800th index (e.g., depth of 800) in varying colors (e.g., gradation), this is a pure example, and the indexes (e.g., depths) from the start point to the end point are not limited to 800 indexes, and the spacing between points per depth is also not limited to the illustrated example. It is also illustrated that the index (e.g., index 0) closer to the start point is darker, and the index (e.g., index 800) closer to the end point is brighter, but this is only to help understanding.

FIG. 12 illustrates edge weights in the tree structure data according to some aspects.

According to some aspects, while generating tree structure data 1230 described above, the electronic device may determine an edge weight 1240 for the edge between the nodes. The edge weight 1240 of the edge connecting the two nodes may be determined based on a distance 1242 (e.g., a point distance) between 3D candidate points indicated by two corresponding nodes, and a distance 1241 (e.g., a line distance) between an epipolar line to the reference point and an epipolar line to the sub-point. The electronic device may determine a sum of the point distance and the line distance as the edge weight 1240. For example, the electronic device may determine the edge weight 1240 for the edge based on the distance 1242 between 3D candidate points corresponding to the two nodes connected to the edge. Furthermore, the electronic device may determine the edge weight 1240 for the edge based on the distance 1241 between the epipolar line to the reference point and the epipolar line to the sub-point corresponding to the 3D candidate point of any one of the two nodes connected to the edge.

The distance 1242 between the 3D candidate points indicated by the two nodes may refer to a distance in a 3D space between a 3D candidate point 1291 indicated by a parent node (e.g., a node of the first depth 1231) and a 3D candidate point 1292 indicated by a child node (e.g., a node of the second depth 1232). The distance 1242 between the 3D candidate points may serve as a weight to alleviate a phenomenon in which the reconstructed 3D shape has a shape that is rapidly bent due to noise.

The distance 1241 between the epipolar lines may be a distance between epipolar lines to two-dimensional points (e.g., reference points and sub-points) used for generating the 3D candidate point 1291 indicated by the parent node. The epipolar lines may represent lines connecting radiation irradiation units 1203-1 and 1203-2 and 3D points generated upon transformation of points (e.g., two-dimensional points) on image planes 1210 and 1220 corresponding to the radiation detection units into a 3D space. For reference, when the 3D center line is projected onto each of the two-dimensional image planes, the distance 1241 between the epipolar lines may serve as a weight to ensure better matching to the two-dimensional center lines in the corresponding two-dimensional image planes.

For reference, an example has been explained above, in which the sum of the distance 1242 between the 3D candidate points and the distance 1241 between the epipolar lines is determined as the edge weight 1240, but aspects are not limited thereto. Each of the edges may have a plurality of edge weights. For example, each of the edges may have a first edge weight indicating a distance between 3D candidate points and a second edge weight indicating a distance between epipolar lines.

FIG. 13 illustrates an example of a 3D path selected using the edge weights of the tree structure data according to some aspects.

According to some aspects, the electronic device may determine a 3D path 1392 by selecting waypoints from among the 3D candidate points between the start point and the end point of the reference center line. The electronic device may determine the 3D path 1392 from the potential paths based on the 3D candidate points, based on a cost calculated using the edge weight of the tree structure data 1330.

For example, the electronic device may generate the 3D path 1392 by selecting a node corresponding to a waypoint from among the nodes of the tree structure data 1330 based on the edge weight described above. The electronic device may determine the 3D path 1392 from potential paths formed by selecting intermediate nodes 1391 between the node (e.g., the starting node) corresponding to the proximal point and the node (e.g., the end node) corresponding to the distal point, using the cost based on the edge weights of the tree structure data 1330. For example, the electronic device may calculate a path cost by summing the edge weights of the edges connecting the nodes that form the potential path for each of the potential paths. The electronic device may determine a path having the lowest calculated path cost (e.g., a path with a minimum cost) among the potential paths as the 3D path 1392. For example, the electronic device may find a shortest path with the smallest sum of edge weights from the proximal to distal points through the Dijkstra's algorithm. The determined 3D path 1392 may represent a center line of the blood vessels in the 3D space, and may also be referred to as a 3D center line.

FIGS. 14 and 15 are provided to explain matching between 3D points and 2D points by pair matching according to some aspects.

The electronic device according to some aspects may pair the waypoints of the 3D center line to the reference points of the reference image and the sub-points of the sub-image. The electronic device may match the determined 3D center line to the two-dimensional center line of the reference image and the two-dimensional center line of the sub-image, respectively.

Referring to FIG. 14, for example, the electronic device may project a 3D center line 1430 onto a reference image. The electronic device may match the indexes of projected points 1431 of the 3D center line 1430 on the reference image and the points (e.g., the reference point 1411) of the 2D center line 1410 of the reference image. Based on the DTW algorithm, the electronic device may determine the index of the reference point 1411 that matches each of the points 1431 of each 3D center line 1430. The DTW algorithm may refer to an algorithm that defines relationship between points by examining the similarity between two curves.

The electronic device may similarly project the 3D center line with respect to the sub-image. The electronic device may match the indexes of the projected points of the 3D center line on the sub-image and the points (e.g., sub-points) of the two-dimensional center line of the sub-image.

Referring to FIG. 15, the electronic device may determine a diameter of the 3D points defining the 3D center line. For example, the electronic device may identify 2D points corresponding to the points on the 3D path in the reference image and the sub-image. The electronic device may calculate a 3D diameter based on the widths of the identified 2D points.

For example, the electronic device may identify a reference point and a sub-point matching the 3D points defining the 3D center line based on the pair matching described above. One or more reference points and one or more sub-points may be matched with each 3D point. Depending on examples, a plurality of 2D points may be matched with one 3D point. The electronic device may obtain diameter information of the 2D points matching a corresponding 3D point. The diameter information may refer to a diameter value of the blood vessels at a corresponding two-dimensional point, and may also be referred to as a blood vessel width. The electronic device may determine the diameter information based on the width at the 2D point. For example, the electronic device may determine the width of the 2D point based on a length along a direction orthogonal to the 2D center line (e.g., reference center line or sub-center line) at a corresponding 2D point in the segmented blood vessel region.

If there are a plurality of 2D points matching the same 3D point, the electronic device may calculate the width at a corresponding 2D point for each of the matching 2D points. For example, the electronic device may determine the widths at the reference point and the sub-point identified for the 3D point. The electronic device may determine a 3D diameter value based on the determined widths.

Referring to the example shown in FIG. 15, the electronic device may backproject a 3D center line 1530 onto a reference image 1510 and a sub-image 1520. The electronic device may map each of the points of the 3D center line 1530 to a point 1515 along the reference center line of the reference image 1510 and to a point 1525 along the sub-center line of the sub-image 1520. The electronic device may calculate a spacing (e.g., a blood vessel width 1591) between an upper contour 1511 and a lower contour 1512 in the reference image 1510. Similarly, the electronic device may calculate a spacing (e.g., a blood vessel width 1592) between an upper contour 1521 and a lower contour 1522 in the sub-image 1520. The electronic device may calculate an average value of the widths of the identified 2D points as a 3D diameter. For example, the electronic device may determine an average value of the determined widths as the 3D diameter value. The electronic device may determine the 3D diameter value for each of the 3D points.

FIG. 16 illustrates reconstruction of a 3D shape according to some aspects.

The electronic device according to some aspects may generate vertices 1620 along a circumference 1610 corresponding to the 3D diameter for each of the points on the 3D path. The electronic device may reconstruct the 3D shape of the blood vessels using a mesh 1630 based on the generated vertices.

The electronic device may generate a circle corresponding to a vessel surface at the corresponding point by using the 3D diameter value determined for each 3D point. For example, the electronic device may generate a circle defining a blood vessel cross section perpendicular to the 3D center line at each 3D point. The electronic device may generate the vertices 1620 corresponding to the circle defining the blood vessel cross section. For example, the electronic device may place the vertices 1620 along the circumference 1610 corresponding to the blood vessel cross section. The electronic device may determine the normal of the surface by calculating the tangent of the 3D center line at the 3D point. The electronic device may generate a circle having the 3D diameter value on its surface based on the determined normal line.

For reference, the diameter information may be obtained in pixel units in the two-dimensional image, and electronic devices may convert the diameter value in the pixel units into a diameter value in length units (e.g., millimeters (mm)) using the calibration factor described above. The electronic device may generate the circle defining the blood vessel cross section at a corresponding 3D point by using the diameter value converted by the length unit. The blood vessel cross section may be perpendicular to the 3D center line (e.g., the blood vessel center line).

The electronic device may convert the vertices 1620 defining the blood vessel cross section generated for a plurality of 3D points into the mesh 1630. The electronic device may convert the vertices 1620 corresponding to the blood vessel cross section into the mesh 1630 through a Poisson surface reconstruction algorithm. The electronic device may generate a face by grouping the vertices 1620 based on the Poisson surface reconstruction. For example, the face may be a triangular primitive and/or a quadrangular primitive. The triangular primitive may be defined and/or formed by three vertices. Similarly, a square primitive may be defined and/or formed by four vertices. The electronic device may reconstruct the 3D shape of the blood vessels by forming the primitive corresponding to the face from the vertices 1620 as described above.

In addition, the electronic device may apply texture information to the primitive corresponding to the face. For example, the electronic device may determine the texture value for any face as a Fractional Flow Reserve (FFR) value corresponding to the position of the blood vessels on that face. The FFR value may be a blood flow pressure value at each position in a cardiovascular system and may be calculated from 3D blood vessels information. Therefore, the electronic device may provide users with more intuitive position-specific FFR values by applying the FFR value for each position in the blood vessels to the primitive as a texture value.

The flowchart and description above are merely examples and may be implemented differently in some examples. For example, in some examples, the order of respective steps may be changed, some steps may be repeatedly performed, some steps may be omitted, or some steps may be added.

The method described above may be provided as a computer program stored in a computer-readable recording medium for execution on a computer. The medium may be a type of medium that continuously stores a program executable by a computer, or temporarily stores the program for execution or download. In addition, the medium may be a variety of recording means or storage means having a single piece of hardware or a combination of several pieces of hardware, and is not limited to a medium that is directly connected to any computer system, and accordingly, may be present on a network in a distributed manner. An example of the medium includes a medium configured to store program instructions, including a magnetic medium such as a hard disk, a floppy disk, and a magnetic tape, an optical medium such as a CD-ROM and a DVD, a magnetic-optical medium such as a floptical disk, and a ROM, a RAM, a flash memory, etc. In addition, other examples of the medium may include an app store that distributes applications, a site that supplies or distributes various software, and a recording medium or a storage medium managed by a server.

The methods, operations, or techniques of the present disclosure may be implemented by various means. For example, these techniques may be implemented in hardware, firmware, software, or a combination thereof. Those skilled in the art will further appreciate that various illustrative logical blocks, modules, circuits, and algorithm steps described in connection with the disclosure herein may be implemented in electronic hardware, computer software, or combinations of both. To clearly illustrate this interchangeability of hardware and software, various illustrative components, blocks, modules, circuits, and steps have been described above generally in terms of their functionality. Whether such a function is implemented as hardware or software varies depending on design requirements imposed on the particular application and the overall system. Those skilled in the art may implement the described functions in varying ways for each particular application, but such implementation should not be interpreted as causing a departure from the scope of the present disclosure.

In a hardware implementation, processing units used to perform the techniques may be implemented in one or more ASICs, DSPs, digital signal processing devices (DSPDs), programmable logic devices (PLDs), field programmable gate arrays (FPGAs), processors, controllers, microcontrollers, microprocessors, electronic devices, other electronic units designed to perform the functions described in the present disclosure, computer, or a combination thereof.

Accordingly, various example logic blocks, modules, and circuits described in connection with the present disclosure may be implemented or performed with general purpose processors, DSPs, ASICs, FPGAs or other programmable logic devices, discrete gate or transistor logic, discrete hardware components, or any combination of those designed to perform the functions described herein. The general purpose processor may be a microprocessor, but in the alternative, the processor may be any related processor, controller, microcontroller, or state machine. The processor may also be implemented as a combination of computing devices, for example, a DSP and microprocessor, a plurality of microprocessors, one or more microprocessors associated with a DSP core, or any other combination of the configurations.

In the implementation using firmware and/or software, the techniques may be implemented with instructions stored on a computer-readable medium, such as random access memory (RAM), read-only memory (ROM), non-volatile random access memory (NVRAM), programmable read-only memory (PROM), erasable programmable read-only memory (EPROM), electrically erasable PROM (EEPROM), flash memory, compact disc (CD), magnetic or optical data storage devices, etc. The instructions may be executable by one or more processors, and may cause the processor(s) to perform certain aspects of the functions described in the present disclosure.

When implemented in software, the techniques may be stored on a computer-readable medium as one or more instructions or codes, or may be transmitted through a computer-readable medium. The computer-readable media include both the computer storage media and the communication media including any medium that facilitates the transmission of a computer program from one place to another. The storage media may also be any available media that may be accessible to a computer. By way of non-limiting example, such a computer-readable medium may include RAM, ROM, EEPROM, CD-ROM or other optical disk storage, magnetic disk storage or other magnetic storage devices, or any other media that can be used to transmit or store desired program code in the form of instructions or data structures and can be accessible to a computer. In addition, any connection is properly referred to as a computer-readable medium.

For example, if the software is sent from a website, server, or other remote sources using coaxial cable, fiber optic cable, twisted pair, digital subscriber line (DSL), or wireless technologies such as infrared, wireless, and microwave, the coaxial cable, the fiber optic cable, the twisted pair, the digital subscriber line, or the wireless technologies such as infrared, wireless, and microwave are included within the definition of the medium. The disks and the discs used herein include CDs, laser disks, optical disks, digital versatile discs (DVDs), floppy disks, and Blu-ray disks, where disks usually magnetically reproduce data, while discs optically reproduce data using a laser. The combinations described above should also be included within the scope of the computer-readable media.

The software module may reside in RAM memory, flash memory, ROM memory, EPROM memory, EEPROM memory, registers, hard disk, removable disk, CD-ROM, or any other form of storage medium known. An exemplary storage medium may be connected to the processor such that the processor may read or write information from or to the storage medium. Alternatively, the storage medium may be integrated into the processor. The processor and the storage medium may exist in the ASIC. The ASIC may exist in the user terminal. Alternatively, the processor and storage medium may exist as separate components in the user terminal.

Although the examples described above have been described as utilizing aspects of the currently disclosed subject matter in one or more standalone computer systems, aspects are not limited thereto, and may be implemented in conjunction with any computing environment, such as a network or distributed computing environment. Furthermore, the aspects of the subject matter in the present disclosure may be implemented in multiple processing chips or apparatus, and storage may be similarly influenced across a plurality of apparatus. Such apparatus may include PCs, network servers, and portable apparatus.

Although the present disclosure has been described in connection with some examples herein, various modifications and changes can be made without departing from the scope of the present disclosure, which can be understood by those skilled in the art to which the present disclosure pertains. In addition, such modifications and changes should be considered within the scope of the claims appended herein.

## Claims

**1.**   A method performed by an electronic device to reconstruct a three-dimensional shape of blood vessels, the method comprising:

acquiring a plurality of medical images captured at a plurality of positions;
generating tree structure data of three-dimensional candidate points corresponding to points along center lines of blood vessel regions of at least two images of the plurality of medical images, wherein the at least two images comprise a reference image and a sub-image;
determining, based on the tree structure data, a three-dimensional path;
determining a three-dimensional diameter for each of points on the three-dimensional path;
reconstructing, based on the determined three-dimensional diameter, a three-dimensional shape of blood vessels corresponding to the three-dimensional path; and
outputting the reconstructed three-dimensional shape of the blood vessels.

**2.**   The method according to claim 1, wherein the generating the tree structure data comprises:
determining a medical image, selected from among the plurality of medical images, as the reference image, wherein the medical image is determined as the reference image based on at least one of a length of a center line of a blood vessel region in each of the plurality of medical images or lesion information of each of the plurality of medical images.

**3.**   The method according to claim 1, wherein the generating the tree structure data comprises:
determining three-dimensional candidate points corresponding to sub-points along a center line of a segmented blood vessel region in the sub-image, for each of reference points along a center line of the segmented blood vessel region in the reference image.

**4.**   The method according to claim 3, wherein the determining the three-dimensional candidate points comprises:

generating nodes corresponding to the three-dimensional candidate points for each depth according to an arrangement order of the reference points along the center line of the segmented blood vessel region in the reference image; and

connecting the generated nodes with an edge.

5. The method according to claim 4, wherein the connecting the generated nodes with the edge comprises:
based on a distance between three-dimensional candidate points corresponding to nodes of adjacent depths of the generated nodes being within a threshold distance, connecting the nodes of the adjacent depths.

6. The method according to claim 4, wherein the connecting the generated nodes with the edge comprises:
based on a distance between three-dimensional candidate points corresponding to two nodes connected to the edge, determining an edge weight for the edge.

7. The method according to claim 4, wherein the connecting the generated nodes with the edge comprises:
based on a distance between an epipolar line to a first reference point and an epipolar line to a first sub-point, determining an edge weight for the edge, wherein each of the first reference point and the first sub-point corresponds to a three-dimensional candidate point of one of two nodes connected to the edge.

8. The method according to claim 1, wherein the determining the three-dimensional path comprises:
determining the three-dimensional path by selecting waypoints from among three-dimensional candidate points between a start point and an end point of the center line of the reference image.

9. The method according to claim 1, wherein the determining the three-dimensional path comprises:
determining, based on edge weights of nodes corresponding to the three-dimensional candidate points in the tree structure data, the three-dimensional path.

10. The method according to claim 1, wherein the determining the three-dimensional path comprises:
based on a cost calculated using an edge weight of the tree structure data, determining the three-dimensional path from potential paths, wherein the potential paths are based on the three-dimensional candidate points.

11. The method according to claim 1, wherein the determining the three-dimensional diameter for each of the points on the three-dimensional path comprises:

identifying two-dimensional points corresponding to points on the three-dimensional path in the reference image and the sub-image; and

determining, based on at least one of widths of the identified two-dimensional points, the three-dimensional diameter.

12. The method according to claim 11, wherein the determining the three-dimensional diameter comprises determining an average value of the widths of the identified two-dimensional points as the three-dimensional diameter.

13. The method according to claim 1, wherein the reconstructing the three-dimensional shape of the blood vessels comprises:

generating vertices along a circumference corresponding to the three-dimensional diameter at each of the points on the three-dimensional path; and

reconstructing, using a mesh that is based on the generated vertices, the three-dimensional shape of the blood vessels.

14. A non-transitory computer-readable recording medium storing instructions that, when executed by one or more processors, cause performance of the method according to claim 1.

15. An electronic device, comprising:

one or more processors; and

memory storing instructions that, when executed by the one or more processors, cause the electronic device to:

acquire a plurality of medical images captured at a plurality of positions;

generate tree structure data of three-dimensional candidate points corresponding to points along center lines of blood vessel regions of at least two images of the plurality of medical images, wherein the at least two images comprise a reference image and a sub-image;

determine, based on the tree structure data, a three-dimensional path;

determine a three-dimensional diameter for each of points on the three-dimensional path;

reconstruct, based on the determined three-dimensional diameter, a three-dimensional shape of blood vessels corresponding to the three-dimensional path; and output the reconstructed three-dimensional shape of the blood vessels.

FIG. 1

FIG. 2

FIG. 3

START

/ 410

ACQUIRE MEDICAL IMAGES

/ 420

GENERATE TREE STRUCTURE DATA OF THREE-DIMENSIONAL CANDIDATE POINTS
CORRESPONDING TO POINTS ALONG CENTER LINES OF BLOOD VESSEL
REGIONS IN REFERENCE IMAGE AND SUB-IMAGE OF MEDICAL IMAGES

/ 430

DETERMINE THREE-DIMENSIONAL PATH BASED ON TREE STRUCTURE DATA

/ 440

DETERMINE THREE-DIMENSIONAL DIAMETER FOR EACH OF POINTS
ON THREE-DIMENSIONAL PATH

/ 450

RECONSTRUCT THREE-DIMENSIONAL SHAPE OF BLOOD VESSEL CORRESPONDING
TO THREE-DIMENSIONAL PATH BASED ON THREE-DIMENSIONAL DIAMETER

END

FIG. 4

FIG. 5

FIG. 6

FIG. 7

FIG. 8

FIG. 9

EP 4 546 266 A1

FIG. 10

1110

FIG. 11

FIG. 12

EP 4 546 266 A1

FIG. 13

FIG. 14

FIG. 15

FIG. 16

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VUKICEVIC ARSO M. ET AL: "Three-dimensional reconstruction and NURBS-based structured meshing of coronary arteries from the conventional X-ray angiography projection images", SCIENTIFIC REPORTS, vol. 8, no. 1, 26 January 2018 (2018-01-26), XP093121821, US ISSN: 2045-2322, DOI: 10.1038/s41598-018-19440-9 Retrieved from the Internet: URL:https://www.nature.com/articles/s41598-018-19440-9.pdf> | 1,3,4, 6-15 | INV. G06T7/593 |
| Y | * pages 3-4, 6-8 * | 2 | |
| A | * figures 4, 5, 6 * | 5 | |
| | - - - - - | | |
| L | VUKICEVIC ARSO M. ET AL: "Three-dimensional reconstruction and NURBS-based structured meshing of coronary arteries from the conventional X-ray angiography projection images - Appendix", SCIENTIFIC REPORTS, vol. 8, no. 1, 26 January 2018 (2018-01-26), XP093257029, US ISSN: 2045-2322, DOI: 10.1038/s41598-018-19440-9 Retrieved from the Internet: URL:https://static-content.springer.com/esm/art:10.1038/s41598-018-19440-9/MediaObjects/41598_2018_19440_MOESM7_ESM.pdf> * page 10, "Algorithm 4" * | 4,6,7,9, 10 | |
| | | | **TECHNICAL FIELDS SEARCHED (IPC)** |
| | | | G06T |
| | - - - - - | | |
| | -/-- | | |

The present search report has been drawn up for all claims

| Place of search | Date of completion of the search | Examiner |
|---|---|---|
| Munich | 7 March 2025 | Kollreider, Klaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 24 20 7754

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| L | VUKICEVIC ARSO M. ET AL: "Computer methods for follow-up study of hemodynamic and disease progression in the stented coronary artery by fusing IVUS and X-ray angiography", MEDICAL AND BIOLOGICAL ENGINEERING AND COMPUTING., vol. 52, no. 6, 27 April 2014 (2014-04-27), pages 539-556, XP093257028, DE ISSN: 0140-0118, DOI: 10.1007/s11517-014-1155-9 Retrieved from the Internet: URL:http://link.springer.com/article/10.1007/s11517-014-1155-9/fulltext.html> * section 2.9 * * page 544, column 2, last par. * ----- | 4,6,7,9,10 | |
| Y | TONG JIJUN ET AL: "The optimization of parameters and matching point pairs in the 3D reconstruction of coronary artery", BIOMEDICAL SIGNAL PROCESSING AND CONTROL, ELSEVIER, AMSTERDAM, NL, vol. 67, 11 March 2021 (2021-03-11), XP086536890, ISSN: 1746-8094, DOI: 10.1016/J.BSPC.2021.102534 [retrieved on 2021-03-11] * section 2.3.2 * ----- | 2 | **TECHNICAL FIELDS SEARCHED (IPC)** |

The present search report has been drawn up for all claims

| Place of search | Date of completion of the search | Examiner |
|---|---|---|
| Munich | 7 March 2025 | Kollreider, Klaus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document